# EUROPEAN PATENT APPLICATION

(11) **EP 4 579 584 A1**
(43) Date of publication of application: **02.07.2025**
(21) Application number: 24175533.9
(22) Date of filing: 13.05.2024
(51) Int. Cl.: G06T 7/00, G06T 7/11, G06T 7/136, G06T 7/62

(54) **METHOD AND SYSTEM FOR AUTOMATIC DETECTION AND MARKING OF THIN-CAP FIBROATHEROMA (TCFA) IN AN OCT IMAGE**

(30) Priority: 29.12.2023 EP 23220784
(71) Applicant: Data Juice Lab sp. z o.o., 03-054 Warszawa (PL)
(72) Inventor: OGONOWSKI, Dominik, 03-054 Warszawa (PL); ROLEDER, Tomasz, 51-649 Wroclaw (PL); KARNY, Michal, 05-805 Otrebusy (PL); DARAKHOVICH, Mikhail, 03-735 Warszawa (PL)
(74) Representative: LDS Lazewski Depo & Partners

(57) **Abstract**

The invention is a method and set-up to detect and mark lipid plaques with thin fibrous cap (TCFA) on Optical Coherence Tomography (OCT) images. Subject of the invention is also a data carrier capable of realizing the invented method.

## Description

### FIELD

The invention is a method and set-up for automatic detection and segmentation of fibrous cap with Thin Cap Fibroatheroma (TCFA) in Optical Coherence Tomography (OCT) images. Subject of the invention is also the device allowing for execution of the aforementioned method.

### BACKGROUND

Cardiovascular diseases are the leading cause of death in developed countries, as well as coinciding with physical disability, which leads to functional limitations for both social and professional life. It is the cause for 16% of global deaths. From 2000 to 2019 there were over 2 million registered cases, with reported numbers going as high as 8.9 million. It has the highest growth amongst the top ten most common causes of death in the world.

Majority of cardiovascular events, such as heart attacks or strokes, are related with development and bursting of injured atherosclerotic plaques. Studies conducted in the last 15 years showed, that there exist multiple types of damaged tissues which, even though they have minimal-to-low tightening of the vessel's lumen, are precursors of coronary thrombosis or cardiac arrest. One of such causes is lipid tissue with Thin Cap Fibroatheroma (TCFA), an anomalous change with large lipid core and a thin fibrous cap separating it from the lumen, which is the cause for -80% of sudden heart-related deaths. TCFA is usually a minimally occlusive tissue, characterized histologically by properties such as: thin fibrous cap (<65 µm), large volume of lipids, and active macrophages near or within the fibrous cap. The highest risk of damages comes from changes localized in regions, where the tension in the walls is high, and where the tissue experiences large biomechanical forces. Rupturing begins a process of coagulation, which can then lead to an acute coronary event.

Detection of tissues at risk of rupturing *in vivo* and the judgement of likelihood of such event were major challenges for clinicians and the scientific community. They were aided with technologies for internal imaging such as Intravascular Ultrasound (IVUS) and Optical Coherence Tomography (OCT). IVUS is commonly used in clinical practices to diagnose and decide on treatments. However, due to limited resolution of -150-200 µm, it is not enough to detect damaged lipid tissues whose thickness is below 65 µm (TCFA). OCT allows for that, as with a resolution of 15-20 µm it can be used to measure cap thickness more accurately. OCT is used to identify anomalous changes and judge their morphology in order to decide on treatment.

Thanks to OCT, it is possible to differentiate between several types of atherosclerotic plaques using OCT images, among which is identification of TCFA. However, it was quickly noticed that the analysis of these images is made difficult by the similar appearance of different types of plaques on the resulting image.

Analysis of OCT images is a time-consuming process that requires highly specialized individuals. Without a precise analysis of the image a doctor cannot request further tests or procedures, which increases risk of a stroke. Additionally, in case of incorrect analysis (human error) - for instance confusing lipid plaque with calcified plaque - can lead to incorrect procedures, which can have negative consequences for the patient. It is even easy to mistake TCFA for regular fibrous cap. Both TCFA and regular fibrous cap have a lipid core, which dissipates light within the OCT and can make it hard to locate the exact boundary of the cap. As the main difference is thickness, uncertainty can easily lead to mistakes.

As a result of this, there have been many attempts at creating a capable automated system for detecting atherosclerotic plaque on OCT images. In majority of studies, they used classification methods in order to classify sets of image data with plaque, without plaque, or with mixed plaque. In other studies, there were also methods made based on a mathematical approach. Some of the works focused on TCFA detection, defining whether or not it exists.

Amongst patented documents, especially noteworthy are the following.

Document US8750615B2 relates to the set-up and the related method of automatic and semi-automatic segmentation and classification of structure and physiology of blood vessels, including segmentation and classification of light, walls, calcified plaques, fibrous caps, metal and bioabsorbable stents, as well as visualization of results. Segmentation of calcified plaques can be used to estimate the distribution of surface calcifications, and a strategy of stenting the area. Additionally, it allows for volumetric segmentation and classification of fibrous caps, which can then provide complex information about mechanisms behind plaque ruptures.

Document EP2903533B1 relates to the set-up, which allows analysis sets of image data by searching for values of many parameters, selected from ones such as: vessel diameter, lumen area, wall thickness, plaque load, vessel remodeling index, tissue type, coagulation size, coagulation location, blood flow, blood pressure, measurement of dynamics of fluids, or types of stent. Image is analyzed in order to automatically calculating a threshold for the value of the parameter, and the level and distribution of pixels is analyzed, for which the median of is used as a threshold value. The data is then visualized in the form of an image on which the pixels where ones below and above the threshold can be marked with distinct colors, or just pixels with are beyond the statistic region are marked with colors.

Document WO2022228463A1 relates to the process and method of judging the state of coronary artery atherosclerotic plaque. Method encapsulates providing an OCT image of coronary artery atherosclerotic plaque for judgement, carrying out identification on the image, and deciding the fiber element based on calculations of thickness of the fiber cap and lipid plaque, as well as judgement of the state of coronary artery atherosclerotic plaque based on the fiber element and the load of the lipid plaque.

Document CN114882017B reveals method and process of detecting thin fibrous caps based on images of intracranial arteries. Method covers obtaining an OCT image of intracranial arteries; segmentation of the fibrous region from the OCT image of intracranial arteries using a trained segmentation model, locating the inner wall of vessel's lumen, capturing OCT image of intracranial arteries according to the inner wall of vessel's lumen and a given distance In order to retrieve the OCT image of intracranial arteries. The method assumes using two trained segmentation models to detect lipid plaque and calcified plaque.

Document US11120548B2 reveals a solution, which simplifies classification of vessel plaque. The solution uses vessel images, on which they mark sections of appropriate cross-sections, after which they compare with at least one characteristic of a classifier, whereas the classifiers refer to classification of fibrous-calcium changes, fibrous-lipid, or others.

### PROBLEM TO SOLVE

Detection of at-risk atherosclerotic plaques *in vivo* and their judgement are still a major challenge for clinicians and the scientific community, largely because the definitions of plaques evolve alongside the growing imaging technology and data processing techniques. Additionally, it is worryingly easy to make a mistake during analysis, which can result in damaging TCFA, which contains a lipid core, thus causing a dangerous release of the lipid contents into the vessel's lumen.

Despite revealing in the technological background a series of automated solutions based on detecting of atherosclerotic plaques, including TCFA, there is still a need for a method of detecting TCFA, which would be both precise and flawless. Such a method must also be trusted by doctors, which must be able to trust the method without needing to double-check it, and thus they must understand it.

Simultaneously, the method should allow quick and easy modification in case of a change in the definition of TCFA in the medical sphere - e.g., in case the thickness when the fibrous cap is considered "thin" changes.

Our method and set-up solve the above issues.

### SOLUTION TO THE PROBLEM AND ITS BENEFITS

The method, according to the invention, allows to use OCT images to automatically detect tissue that is at risk of rupturing (TCFA) using mathematical methods (algorithms). Image containing segmented lipid plaque is processed via mathematical algorithms, which then detect whether a given analyzed lipid plaque contains TCFA or not.

Such combination of mathematical methods allows for considerable shortening of the time needed to analyze OCT images to locate TCFA, thus allowing for an instantaneous recognition by the leading doctor. Time for complete automatic analysis of an OCT image of a vessel in the length between 50 and 70 mm using the invented method is <10 minutes. For comparison, a human would need to analyze 300 frames (a full scan has around 450 frames) for around an hour. We also eliminate human error.

Invented method allows for high accuracy of automatic detection of TCFA on an OCT image; while considering artifacts and branching vessels, even though large branches (>2 mm) create an increase in lumen area, and artifacts limit the IR light. Additionally, the invented method allows high accuracy of differentiating fibrous cap from structures such as macrophages or cholesterol crystals, which have a similar intensity on OCT images.

On top of that, thanks to using a mathematical algorithm in the most crucial point of locating TCFA - i.e., during separation from lipid plaque - it allows for better understanding of the result shown by the image. This will lead to the invention being more trustworthy for doctors wanting a solution, as they can more easily understand how the solution was formed.

Currently used solutions using machine learning are "black boxes." After training the algorithm, it is hard to say why it outputs a certain image, which makes it more difficult to solve disputes, when the machine shows a different result to what the doctor would expect. Classical algorithms (such as the ones used in the following invention to detect TCFA) are more rigid and are meant to reflect the detection steps used by doctors during manual analysis. This means that if there is a problem, it is usually easier to see and explain. Classical methods also allow to better understand how to detect fibrous cap and could even allow for a better understand and definition of TCFA, allowing for an insight into the logic of differentiating TCFA from other structures.

The difficulties in creating a proper mathematical algorithm come from the fact, that simplifying what doctors do into classical algorithms is usually quite complicated. The invention however has manager to find a way that allows for 76-90% accuracy in TCFA detection.

Additionally, the invented method allows for its easy modification to adjust to the changing definition of how thick a fibrous cap must be to qualify as TCFA. Depending on their needs, a doctor can even manually input a thickness for the algorithm, and it will treat it as the threshold value. Modification within this variant is as simple as inputting the proper value with the user interface.

In contrast to this, in case of a change in definition of TCFA a machine learning model might need a complete retraining of their model. This means that the proposed invention has an added benefit in terms of its elasticity, ease, and speed of modification; without a need to engage a whole team of data analysts.

Another benefit of the invention is that it allows for judgement of quality of the vessel wall by looking for TCFA both online (e.g., through an uninterrupted stream of data coming from an OCT device), or offline. In offline the invention is used as a helpful diagnostic tool, while in online mode it is a tool helping the assisting doctor (e.g., an interventional cardiologist) for instance during simulations of clearing out coronary arteries. The second usage can have significant importance for training residents without risking patients - for example when learning on phantoms. It is also possible to implement the method in real time using an Augmented Reality system (AR), which allows for visualization of TCFA in a more convenient way for doctors, residents, and students.

### SHORT DESCRIPTIONS OF FIGURES

Subject of the invention was schematically represented on the images. Examples of executing the invention are going to be describe further alongside said figures, on which digits mark the same elements.
Figure 1 shows an OCT image before segmentation of lumen (left panel) and after segmentation of lumen (right panel). The gray area on the right panel highlights the segment classified as lumen light.
Figure 2 shows the OCT images for subsequent frames before segmentation of lumen (top panel) and after segmentation of lumen (bottom panel) in the case where branching of the Bessel is visible on the image. Dark gray area on the bottom panel highlights an image segment classified as lumen 1. On the bottom panel light gray color is also used for bifurcation 2.
Figure 3 shows an OCT image with a visible probe * and lipid plaque 3.
Figure 4 shows an OCT image before segmentation of lumen (left panel) and after segmentation of lumen (right panel). On the right panel the segmented lumen is marked 1 and the lipid angle α marking the borders of the lipid plaque 3.
Figure 5 shows a diagram of how "Triangle Thresholding" algorithm works.
Figure 6 shows a top-level overview of how the invention operated its fibrous cap detection for different weights for the "Gradient Guessing" and "Triangle Thresholding" algorithms.

### DEFINITIONS USED IN THE INVENTION

By "atherosclerotic plaque with thin fibrous cap", "at-risk plaque", or "TCFA" we will mean a lipid-rich atherosclerotic plaque with a thin fibrous cap, which can easily rupture, leading to spillage of lipids into the vessel. It is important to underline, that TCFA does not have a permanent definition, however it is usually defined as a lipid-rich plaque whose fibrous cap thickness is <65 µm. The value of <65 µm, for the purposes of this invention, should not be seen as a limitation, as the invention provides means of adjusting the thickness threshold were the definition of TCFA to change within the medical field.

In rare cases TCFA may also be defined using the lipid angle. In such case, for lipid plaque to be considered a TCFA it must have minimal cap thickness below a pre-defined value, such as 65 µm, and the lipid angle must be above 90 degrees, for example above 120 degrees.

"Fibrous cover" is the layer between a lipid and lumen, is in this document also referred to as "cap" or "fibrous cap".

"Vessel light" is also referred to as "lumen".

The terms "lipid" and "lipid plaque" are used interchangeably, and for the purposes of this invention have the same meaning, understood well within the field.

By the term "image from OCT imaging" we understand both the images, as well as videos captured using OCT. In the case of videos, the invention treats them as a series of images, each frame being a single OCT image, here understood as "frames" or "(OCT) images".

The term "catheter artifact" refers to a specific artifact caused by the imaging method, in which on the image a very bright ring is visible. This is a result of a long scan (timewise) or a short one (distance-wise) due to which the probe re-entered the catheter during the scan and attempted to image inside of it.

The term "brightest pixel" has the purpose of referring to the pixel closest to white in a set of pixels, and the term "darkest pixel" refers to the pixel that is closest to black in a given set.

### DETAILED INVENTION DESCRIPTION

The description that follows is intended as an example, and not for the purpose of limiting the score, capabilities, or configuration of the invention in any shape or form. The description below includes a handful of practical illustrations relating to the realization of the invention. Someone with experience in the field will notice, that many of the mentioned examples can have multiple alternatives, which they would be able to modify with ease. Such modifications are also within the boundaries of the invention.

The invented method uses an intravascular OCT scan with at least single frame with lipid plaque as data input. Detection of lipid plaque on the OCT image is ideally realized via a machine learning model trained to find lipid plaques. Alternatively, the lipid plaque can be detected and segmented manually by an expert in the field.

### Receiving input data for the invention

### A. Receiving a model for detecting lipids.

Basic scope of the invention does not reach the process for training the method of detecting lipids. The invention can use any available method for lipid detection, using tools such as (but not limited to) Machine Learning or Artificial Intelligence; trained on OCT images to detect lipid plaque.

Creating of such a model has the following steps:
- downloading medical data of OCT images using Intravascular Optical Coherence Tomography (IVOCT) and labeling the data with labels depicting the locations of lipid plaques. It is beneficial if these images are of varying quality, as well as include different shapes, distributions, and morphology of TCFA within the fibrous tissue.
- training of the model using a neural network on the labeled images, in order to create a model that can detect lipid plaques.

Such a model would ideally have a high accuracy in plaque detection, such as ideally having 90% or more, such as 95%, and even better 99%. One can also use models of lower accuracy of detection of lipid plaques, such as models of accuracy below 90%, such as 80% or less, however such models are less than ideal, as the lower accuracy of detection of lipid plaques will then contribute to lower accuracy of TCFA detection using the invention.

Similarly, such a model should ideally also provide a segmentation of lumen with as high accuracy as possible such as ideally having 90% or more, such as 95%, and even better 99%. One can also use models of lower accuracy of detection of lumen, such as models of accuracy below 90%, such as 80% or less, however such models are less than ideal, as the lower accuracy of detection of lumen will then contribute to lower accuracy of TCFA detection using the invention, due to potential errors with defining the thickness of the fibrous cap. Ideally, the model should be able to provide a correct segmentation of lumen even when the image has visible bifurcations.

Because the creators of this invention during their research have created and trained a model for lipid detection, which combined with the invented method for TCFA detection allows for exceptionally high accuracy of TCFA detection, below with the title of non-limiting, especially useful example of training a model to find lipids, a method for obtaining such a model will be presented:
i) Downloading medical data obtained using OCT imagery

In the first step, OCT image data is downloaded by the model.

### ii) Removing watermarks and other text elements

OCT image data are stripped of any markers (watermarks, texts), contours, measurements (texts). If the data contained the above objects, it was removed by an appropriate algorithm, designed with the express purpose of unification of input data. Creation of such an algorithm is within the capabilities of someone knowledgeable in the field and is not part of the invention. Alternatively, such objects can be removed manually by the operator, however this prolongs the analysis time, and is thus undesirable.

### iii) Initial review of data

Next up, the data (stack of scans - vertical slices of the coronary vessel - known as a pullback) is initially processed. Initial processing relates to checking whether a given vessel contains anomalous plaques and labeling where in the morphological structure they are present, whether stent is present, or artifacts, and if so, then labeling the artifact type. If analysis of the data was possible (i.e., the quality of the OCT recording was high enough), then the material was used in the dataset, which acts as the foundation for training lipid detection models. In the cases of controversial data, OCT image analysis experts were consulted.

### iv) Segmentation and manual analysis

Following that, the data was passed to manual segmentation. In order to ensure replicability of the finished analysis, there were instructions prepared as well as documentation describing the structure of OCT images.

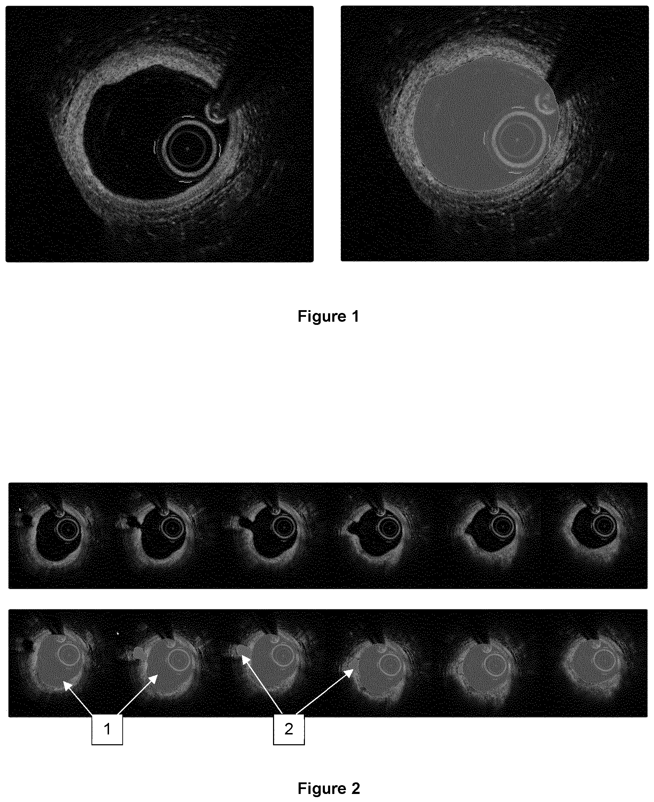

In the instructions for manual segmentation the following segments were marked for manual segmentation:
- lumen contour 1 (see Figure 1), with separately marked bifurcations 2 of the vessel, if they were present (see Figure 2).
- probe contour, even if the probe * was adjacent to a vessel wall.

Manual segmentation was done on every vertical slice of the OCT scan (from 200 to 531 slices per scan).

Within the manual analysis, artifacts were marked within the analyzed slices of OCT scans. Artifacts were divided into three categories:
- artifacts caused by the light propagation due to its physical properties, within the catheter, lumen, and vessel wall
- artifacts caused by the position and motion of the catheter (probe)
- artifacts caused by the presence of a meta stent, which block penetration of light, leaving behind characteristic shadows.

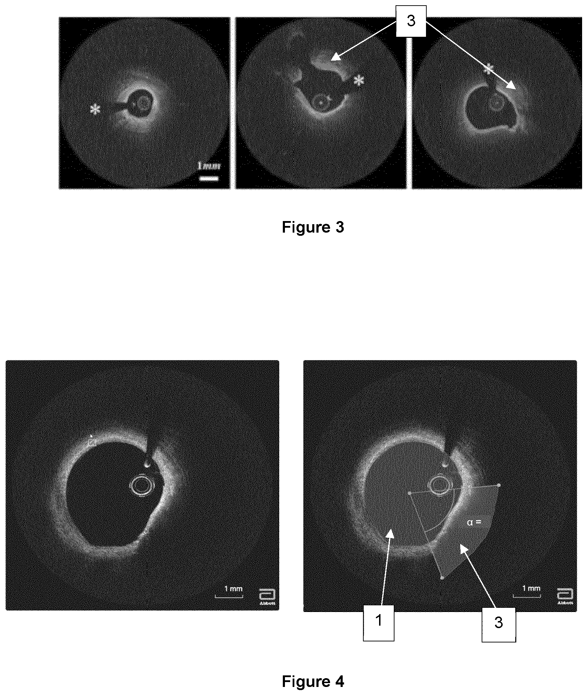

With that data segmentation of lipid plaque was done. Lipid plaque 3 in OCT images is defined as an atherosclerotic plaque with areas of low signal intensity and diffused borders (see Figure 3). Side borders of lipids are defined by the lipid angle α, i.e., the angle whose arms define the lipid borders (see Figure 4). Within these segmentations along the entire lipid angle and to the depth of 1.5 mm into the vessel wall, is marked as lipid plaque.

Based on the established data, a model was made for automatic detection and segmentation of lumen, as well as a model for automatic detection and segmentation of lipid plaque.

### v) Picking model architecture and training the model

A UNET model was chosen, i.e., a convolutional neural network for object segmentation on an image (CNN). Main parameters of the model are as follows: activation function: Relu; loss function: mloULoss; optimization: Adam. Alternatively, one can use other architectures of deep neural networks such as DeepLab, FastFCN, or transformer-type architectures.

Supervised segmentation was used for training a deep neural network, with exceptional consideration for the used convolution operators. Optimalization was done towards maximizing IoU/Dice for lipid segments.

Augmentation of data was done, i.e., the training dataset was constantly expanded with newly generated data.

An attenuation function was used.

### vi) Model validation - detection of false positive segments

A comparison analysis was done between sets of images. Comparing images allows to answer the question whether an analyzed image is more similar to correct or false positive images. A method was chosen using deep convoluted neural networks using Deep Ranking architecture. Benefits of such a solution is a training process based on positive and negative examples of images.

In the analysis predictions from previously trained segmentations models were used, and based on them examples were made for false positive and correct predictions (with preset characteristics vector). After choosing the characteristics vector for a given image, it is compared with the example sets. The set of distances from models based on several types of images and of example images, is further analyzed with unsupervised segmentation - within the used solution: using kNN. An alternative method is comparison of multi-dimensional distance matrices and defining custom decision thresholds for whether a certain image is correct or a false positive.

An additional safeguard from false positive results was an extra classifier based on a neural network, meant to detect whether a given frame has anomalous plaque or not. ResNet architecture was used here, optimized for lipid plaque.

Frames were removed, when result of both models had probabilities of less than 30 percentile from the validation set.

### vii) Achieved results

Dice Coefficient 75.93%
Top 1 Accuracy 98.99% (measured by area)

Calculation time with 4 NVIDIA V100 GPUs (cumulatively 64GB RAM) is <7 minutes for qualitative analysis and <1 minute for quantitative analysis.

### viii) Summary

As mentioned above, the described method of creating a lipid detecting model is purely an example. Someone knowledgeable in the field can easily modify this method, pick different parameters, or use a custom model based on their own knowledge. In the Background for this invention there are models for detecting lipids (lipid plaques), which can be used interchangeably with the model described above.

Basic aim of the method has to be providing the highest possible accuracy of lipid and lumen detection from the OCT image, as this provides a better accuracy of TCFA detection at later stages.

The least useful, though also a possible method for the invented detection of TCFA, is for example using manual detection and segmentation of lipids, however this significantly increases analysis time, and is affected by human error.

### B. Detection of lipids on an OCT image, which is to be analyzed

First four methods for detecting lipids (i-iv) generally relate to the first four steps in point **"A.** Receiving a model for detecting lipids." And area meant to fit the OCT image for the purposes of the model. For the sake of brevity, they will not be described in detail.
i) downloading medical data obtained with OCT imaging, which are to be used in the analysis,
ii) detecting lumen with classical algorithms,
iii) removing watermarks and other texts,
iv) manual segmentation and analysis,
   At this stage, frames with catheter artifacts are detected and deleted.
v) creating lipid predictions with the pre-trained UNET model Within this stage before starting the prediction the images might be transformed from cartesian coordinates to polar coordinates, and after prediction transformed back, if this is required by the trained model.
vi) filtering of the results and deleting of tiny (i.e., of surface area less than 20 pixels) lipid segments
vii) marking the lipid angle 1.5 mm into the vessel wall.
viii) rejecting lipids classified as False-Positive (FP) using a method for comparing images (Deep Ranking) with two trained models which work on several types of images:
   ▪ Every lipid segment detected by the UNET model was compared with a previously collected database of TP (True-Positive) and FP Segments.
   ▪ If a segment was considered as more similar to FP than TP, then it gets removed from the set of segmented lipids.
ix) marking the detected lipids within the set of images.

### The Invented Method

Steps below are part of the invention described within this document and will be described in detail alongside specific usage examples.

The subject of the invention is an automatic method for detecting and segmenting Thin-Cap Fibroatheroma (TCFA) on images obtained using Optical Coherence Tomography (OCT). Such detecting and segmenting of TCFA happens fully automatically i.e., with no human input. The invention includes the following steps:
a) downloading of OCT image data which are to be analyzed in order to detect and segment TCFA, where such data contains at least one frame, on which at least one lipid plaque is present and labeled as a segment to analyze.

Downloading OCT image data can be done, for example, through a direct stream of data from an on-going OCT examination into a unit capable of detecting and segmenting lipid plaque, and then directly into a unit capable of executing the invented method (on-line mode). Alternatively, it can also be done on saved, complete data from previously conducted OCT examinations, which then were passed through a unit capable of detecting and segmenting lipid plaques (off-line mode).

Additionally both in the case of on-line and off-line scans, OCT image data can be preselected in order to limit the input data solely to those images, which contain lipid plaque. In this case after detecting and segmenting lipid plaques, the unit set to realize the invented method is only provided with those OCT images, on which lipid plaque was located.

Data from OCT imaging can be in any format compatible with the invention. Format of the data is not limited by the invention - according to the aforementioned definition, those can be both image formats as well as video formats, which are treated by the invention as a series of singular, subsequent images.

Data from OCT imaging must contain at least one image, on which there is at least one lipid plaque present. Lack of lipid plaques on an image can be caused by an incorrect segmentation, or simply mean that the patient has no lipid plaques, and in the second case that would also mean a lack of TCFA.

The at least one aforementioned lipid plaque is marked as a segment to analyze. "Marked as a segment to analyze" is to be understood as any marking of lipid plaque in a way that allows identification by the invented method. Lipid plaque must be marked in an appropriate manner to be identified and analyzed in order to detect and mark TCFA according to the invention. Such marking does not need to be recognizable visually, but it can be. An example of a marking recognizable visually is marking it with a color or some other marker. Alternatively, lipid plaque can be surrounded with a continuous line along its contour, for instance when the lipid was marked manually by an expert in the field. In the best-case scenario, the lipid plaque is marked via a lipid angle.

Preferably lipid is marked in a digital manner, allowing for digital identification within the invention. As an example of a preferable segmentation one can use representing an OCT frame as a series of characters, which allow the processor, or an analogous unit, to identify the lipid angle defining the lipid plaque in a given frame, which is to be analyzed.
b) Calculating approximate position of the fibrous cap border independently by two algorithms: "Gradient Guessing" and "Triangle Thresholding"; within the aforementioned at least one lipid plaque marked as a segment to analyze, and executed within each light rays within the image, and then calculating a weighted average for the two aforementioned algorithms.

The term "independent" working of algorithms does not mean that the algorithms cannot be done simultaneously. It simply means that each of the algorithms works on the given OCT data, without combining the results of the algorithms on this stage. Algorithms can be executed in any order, one after the other i.e., in sequence. Alternatively, algorithms can also be executed simultaneously.

Algorithms are executed alongside light rays within the image (also known as A-line(s)), and for each ray that contains at least one lipid plaque segment marked for analysis.

"Gradient Guessing" algorithm takes the following steps:
localization of the brightest pixel for every A-line, which is marked as the maximum light intensity for a given A-line,
localization of a series of neighboring pixels, laid out deeper than the brightest pixel, for which the light intensity level is from 65 to 85%, ideally from 70 to 80%, compared to the maximum light intensity for a given A-line,
marking the center of the aforementioned series of pixels as a first potential spot for the fibrous cap boundary

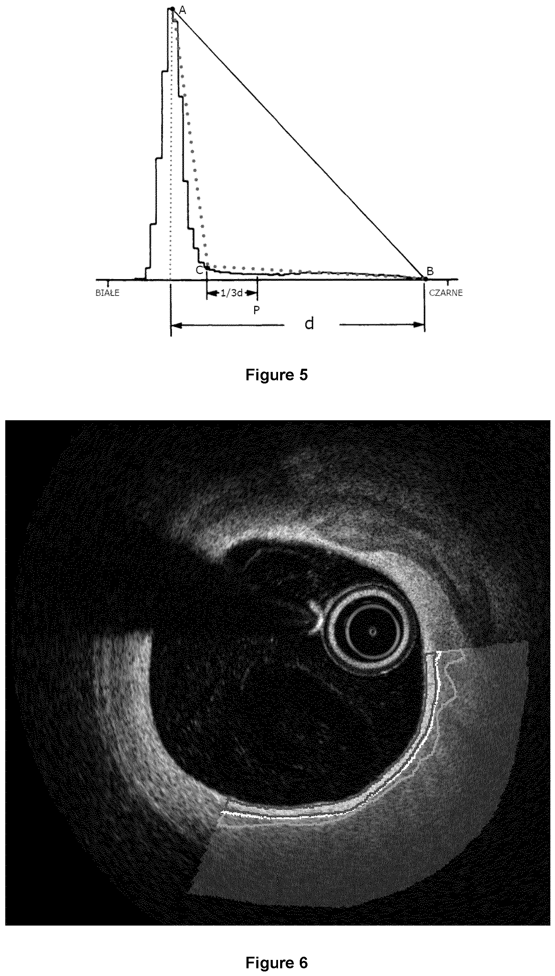

Working of the algorithm "Triangle Thresholding" was presented schematically on Figure 5. Algorithm this corresponds to an algorithm revealed in the publication "Automatic measurement of sister chromatid exchange frequency.": written by G. W. Zack, W. E. Rogers, and S. A. Latt (Journal of Histochemistry & Cytochemistry 1977 25:7, 741-753). This algorithm is made of the following steps:
receiving a histogram of pixel intensities for a given OCT image,
marking two points on the histogram: first point A which is the top of the highest peak, and point B representing the value of the darkest pixel,
marking third point C on the histogram, existing between aforementioned points, so that the surface area of the ABC triangle is maximized,
defining a distance d between the crossing of the OX axis of the histogram from lines that are perpendicular to the OX axis and pass through point A (first line) and point B (second line),
defining a third crossing point on the OX axis of a line passing through point C, perpendicularly to the OX axis,
defining a point P which exists one third of the distance d from the crossing point of the third line with OX axis, and treating the value of such point as a threshold value,
marking pixels on the OCT image, for which the intensity values are above the threshold value as a second potential location for location of the fibrous cap.

In the process of executing the above algorithms we receive the first two approximations of where the fibrous cap exists within the lipid - marked here as the first and second potential locations for fibrous cap border. The terms "first" and "second" have no significance on the order of the algorithms. They are merely used to signify, that the results are separate, unrelated approximation results.

Next up a weighted average is calculated using the results of the two algorithms. In order to calculate the weighted sum, the following range of weight ratios is used, where the weights are decided in such a way, so that they sum to 1: from 0.60 to 0.90, and ideally from 0.65 to 0.85, for the "Gradient Guessing" algorithm, and from 0.10 to 0.40, and ideally from 0.15 to 0.25, for the "Triangle Thresholding" algorithm. In most ideal variant the weights are 0.75 for the "Gradient Guessing" algorithm, and 0.25 for the "Triangle Thresholding" algorithm.

Example results for three different ratios of weights are presented on Figure 6 and described in more detail in Example 4.

During execution of this stage we reach an approximate location for the fibrous cap border.
c) Smoothing of the calculated boundary of the fibrous cap within the aforementioned at least one lipid plaque marked as a segment to analyze.

Smoothing of the image aims to provide a cleaner, final boundary of the fibrous cap within the aforementioned at least one lipid plaque marked as a segment to analyze.

The invention is not limited to a specific method of smoothing, and any method known in the field can be used, such as a uniform, triangle, mean, or Gauss filters.

Ideally a Savitzky-Golay filter is used. In this variant used within the invention, there are the following smoothing steps:
if the number of data points is larger than 60 data points (i.e., lipid angle of at least 36 degrees), then the used window is of size 36,
if the number of data points is between 24 and 60 (i.e., the lipid angle has between 14.4 and 36 degrees), then the used window is of size 24,
if the number of data points is between 4 and 24 (i.e., lipid angle has between 1.8 and 14.4 degrees), the window size is equal to the number of data points, and
If the number of data points is less than or equal 3 data points (i.e., lipid angle has at most 1.8 degrees) then no smoothing is done.
d) detecting the thickness of the fibrous cap by locating the lowest distance between the cap border and lumen edge.

To decide the thickness of a fibrous cap one measures the shortest distance between the border of the cap and the (blood)vessel edge. Edge of the vessel, also referred to as lumen edge, is the touching point of lipid plaque 3 with lumen 1 across the entire length of the lipid angle (α) (see Figure 4).

Method of doing such a measurement would be obvious for anyone knowledgeable in the field, and can be done, for example, with use of another algorithm or via direct measurement on the imaged frame.
e) defining whether a given lipid plaque is TCFA or not, and labeling it as TCFA if the definition result is positive.

Defining whether a lipid plaque is TCFA or not is done via the thickness of the fibrous cap defined in stage d). Lipid plaque is defined as a TCFA, when the thickness of the fibrous cap does not exceed the threshold value a threshold value. If the thickness is always above said threshold value, then the lipid plaque is not a TCFA. The result of deciding whether a given plaques contains TCFA or not is seen as positive, when it is decided that the lipid plaque contains TCFA. Otherwise, the result is seen as negative, and the lipid plaque is not marked as TCFA.

In one of the variants of the invention, the given threshold value for fibrous cap is inputted beforehand into the unit (such as a processor) which realizes the invention. In such a case, this value should reflect the current definition of TCFA thickness in the medical field. On the priority and filing date of this invention this threshold value is equal to 65 µm, which means that lipid plaques with caps of thickness equal to or less than that value are defined as TCFA.

In a more useful variation of the invention, the given thickness threshold for the fibrous cap is inputted manually by the user in accordance with their preference, before using the invention. This allows the user to adjust the parameters - for instance based on the surgical skills of a doctor- detection of TCFA. While conducting intravascular surgeries, it is necessary to remove the calcified plaques without damaging lipid plaques, within which TCFA provides a considerable risk due to the thin fibrous cap, susceptible to rupturing due to damage caused by the catheter. A more experienced surgeon will be able to maneuver the catheter without risk for such plaque, even with lower threshold values for fibrous cap thickness, and would only be warned about TCFA for exceptionally thin caps. Meanwhile a less experienced surgeon could input a larger value, resulting in more detections of TCFA, but increasing the safety of the patient.

Optionally, in the case where on the OCT image data the aforementioned at least one lipid plaque is marked by a lipid angle, an extra condition can be added to define whether a lipid plaque is a TCFA. Such a condition is dependent on the TCFA definition used in the medical field, which in some cases includes the lipid angle, above which the lipid plaque can be labeled as TCFA. In the invention it is possible to consider such threshold value for the lipid angle during analysis. In such a case, TCFA is present when the thickness of the fibrous cap does not exceed the given value, and when the aforementioned lipid angle is higher than the wanted angle threshold. For example, a lipid angle threshold may be above 90 degrees, ideally above 120 degrees.

Such a given threshold value for the lipid angle can be inputted analogically to the threshold value for fibrous cap thickness, leading to analogous benefits.

Marking of the lipid plaque as TCFA in a variant of the invention is done in such a way, so that the TCFA can be seen visually by a person further analyzing the OCT images. Such a method encapsulates, without limitation, hatching, shading, or coloring TCFA, marking TCFA or the border with a marker, such as an arrow, text, or a number; surrounding TCFA with a continuous line delimiting its contour; or marking the lumen edge in locations of TCFA, for instance with a color or a thicker border.

In the most beneficial variant of the invention, the method allowing for visual recognition of TCFA is done via layering the OCT images, including the results of detecting and marking of TCFA, reconstructing the tissue directly in augmented reality (AR) and/or mixed reality (MR). Reconstruction in this context means transforming the OCT images into three-dimensional form, which can then be displayed in an AR and/or MR system. Example implementation of such a variant is described below in "Devices allowing for realization of the invented method".

In the case where OCT image data has more than one lipid plaque, the method of the invention applies the stages b)-e) to each of the lipid plaques. These stages can be executed in sequence for each plaque, or in parallel for all of them.

### Set-up according to the invention

The elements below were described in detail within the invented method, and for the sake of brevity will not be described in precise detail again. Optionally, information below can be read as additional specification for the invented methods with properties characteristic of the invented set-up, which can also relate to the invented method.

Subject of the invention is also the set-up for automatically detecting and labeling TCFA on Optical Coherence Tomography (OCT) images. Such set-up has at least one processor configured to:
a) download OCT image data, which are to be analyzed in order to locate and mark TCFA, containing at least one image, on which there is at least one lipid plaque, where the aforementioned at least one lipid plaque is marked as a segment for analysis.
   Such data must have a proper format, so that the processor is able to read the data as a series of images (frames).
b) calculating an approximate location of the fibrous cap border via independent execution of two algorithms: "Gradient Guessing" and "Triangle Thresholding"; within the aforementioned at least one lipid plaque marked as a segment to analyze, along each of the light rays,
   and then calculating the weighted average of the results for the two aforementioned algorithms.
c) smoothing of the image with the resulting fibrous cap border within the aforementioned at least one lipid plaque marked for analysis.
d) Deciding the thickness of the cap via measuring the shortest distance between the fibrous cap border and the lumen edge.

Measuring the thickness of the cap can be done with any method, trivial to an experienced user.

In the ideal variant of this invention, the processor realizes an algorithm designed explicitly for this purpose. Known are numerous tools allowing measuring of distances on images, which can be used for this purpose.
e) Defining whether a given lipid plaque is a TCFA or not, and marking it as TCFA when the result is positive.

In the cases where in the data from OCT imaging more than one lipid plaque is present, the processor is additionally configured to conduct stages b)-e) for each of the lipid plaques sequentially (i.e., one after the other) or in parallel for all plaques at once.

In the ideal variant of this invention, the invented set-up contains at least one user interface configured to allow inputting into the processor mentioned in step e) threshold values for fibrous cap thickness. Such an interface may include keyboard, mouse, electrical device such as tablet or smartphone, as well as any other device allowing inputting of information to the processor from the user.

In a different, ideal variant of the invention, which can be used separately or in addition to the above variant, the set-up according to the invention contains at least one imaging module configured to display the segmentation results of detected TCFA plaques. Such a module can be any screen, such as a monitor, 2D project, device allowing for 3D visualization, AR or VR headsets, and any other device, which allows the user to visually observe.

### Memory storage according to the invention

Part of the invention is also memory storage, which contains the computer software which instructs the processor with execution of the above-described steps from this document, of detecting and segmenting TCFA on Optical Coherence Tomography (OCT) images.

### Devices allowing for realization of the invented method

Describe below are example devices capable of an exemplary realization of the invention.

### • Application installed on a computer

Thanks to a computer application (application that can be installed on a computer) a doctor can have easy access to visualization of the blood vessel and direct access to the segmentation of TCFA plaques on the image. Plaques can be marked with arrows, colors, shading and any other method allowing for visual identification by the doctor.

Computer application also allows for easy modification of TCFA definition via inserting of the preferred threshold value in the correct field in the application.

Analogous application, allowing exact or similar functions, can also be installed on a tablet, phone, or another device equipped at least with a module allowing for visualization of the vessel, and a processor and operating memory allowing for operation of the application.
• In an ideal variant, the application works in conjunction with an external device ("add-on"), connected to an OCT device and capable of downloading image scans from it, and sending them to the computer application. Such scans are then preprocessed in order to detect the lumen and segment lipid. Afterwards, the segmented lipids function as a basis for the invented method. Headset allowing presentation in mixed or augmented reality

It is possible to visualize and layer reconstructed images of coronary vessels obtained in real time and executed analysis directly onto the oculars - the field of view of the operator in case of mixed reality. Such imagining in mixed reality can allow interprocedural monitoring of a surgery. It is then completely adjusted to the anatomy of the patient and the intervention happening, and allows for a more complete understanding of both the possible results of an intervention, as well as making it easier to select a device and procedural technique without pausing the surgery, thanks to the constant flow of information regarding at-risk tissue. It is also possible to control the image and information using voice commands.

Analogically, in case the oculars are to be used in training new doctors, it is possible to teach the process of a surgery on special phantoms of blood vessels, using a headset for presentation in mixed reality.

It is also possible to present the virtual image of reality in place of mixed reality. In such a case TCFA analysis is realized on a fake, generated for the sake of the learning future doctors, image of a blood vessel, or on a real blood vessel image coming from a previously saved database of OCT images.

The created application using mixed or virtual reality alongside deciding a personalized tutoring path allows for not invading the patient while teaching about how to correctly perform surgeries based on OCT images.

The method of presenting locations of TCFA can be adjusted to the preferences of the user, ideally without disturbing the area marked as TCFA with an arrow. Alternatively, the OCT images, on which TCFA is present, can be augmented with a thicker and/or colored vessel wall wherever TCFA plaques was detected. It is also possible to implement TCFA marking in a different way, such as applying a color and/or highlights on the plaque identified as TCFA within this method.
• Holograms allowing presentation of the blood vessel in 3D

As an alternative solution, allowing for presentation of vessel images alongside TCFA one can propose presenting of aforementioned vessel in three dimensions (3D) using holograms. The method of displaying TCFA can be then analogous to that on a computer monitor or headsets, but within actual 3D space.

### Using the invention

The invention described will have medical uses.

It can be used as aid for doctors (such as cardiologist or angiologist) during diagnosis. In the case of interventional cardiologist, and other doctors conducting cardiovascular surgeries, it can also be a useful tool, allowing for avoidance of easy-to-damage TCFA.

Another use of the invention is using in education of students and residents, which are not as experienced, so that they are able to independently analyze OCT images. Thanks to the invented method they would be capable of quickly verifying their own assumptions about the specific type of plaque, and since the method will provide them with high certainty whether the plaque is TCFA or not, independently of the complexity of the vessel's geometry.

Both of the above uses are especially interesting in case of using the invention within devices operating in mixed reality, as described above.

### EXAMPLES

Invented method, divided into stages, is shown in detail in execution examples below.

### Example 1 - Training a model to detect lipids

Downloaded image data relating to the inside of a blood vessel, obtained using OCT technology.

Using the above OCT images with the help of doctors, a dataset was created with labels marking locations of lipid plaques and vessel lumen.

Images were pre-processed, during which:
**i.** Watermarks and other texts were removed.
**ii.** Dataset was transformed from cartesian to polar coordinates.

Training of the Unet 2D model:
i. Neural Network was trained on images in polar coordinates, which contained at least two segments (lumen, lipids) using an attenuation function.
ii. Main model parameters used were as follows:
   - Activation function: Relu
   - Loss function: mloULoss
   - Optimizer: Adam.

With each added image the data was augmented, i.e., the training dataset size was increased by adding new, generated data.

Model validation was conducted during which the model was confirmed as a valid method of detecting lipids. On the whole dataset the Dice coefficient was equal 50.4, while on lipid-containing frames it was equal to 69.92.

### Example 2 - Detecting lipids on OCT images using a model trained in example 1

Downloaded OCT image data, which is to be analyzed, and removed watermarks and other texts from it.

Detected lumen using classical algorithms. To do this, a histogram correction was made (CLAHE) as well as a Gauss filter applied. Afterwards, artifacts were removed. The image was binarized using the Otsu method, after which it was transformed with morphological operations. Transformation to polar and cartesian coordinates was used. Next up the contours were detected and combined into a single object, based on which the final contour was defined, which corresponds to lumen.

In order to detect lipids, the image was preprocessed during which:
i. Frames containing the catheter artifact were detected and removed.
ii. Image was transformed from cartesian to polar coordinates.

Detection of lipids was conducted using the pre-trained Unet model from example 1.

The image was passed to postprocessing, during which:
i. The image was transformed from polar to cartesian coordinates.
ii. Smaller (i.e., of surface area of less than 20 pixels) lipid segments were filtered and removed.
iii. Lipid angle was marked 1.5 mm into the vessel wall.
iv. Rejection of lipids classified as False-Positives (FP) using a method leveraging similarity of images (Deep Ranking) using two trained models, working on several types of images:
   ▪ Every lipid segment detected by the Unet model (described in example 1) is compared with a database of pre-collected segments containing FP and TP (True-Positive).
   ▪ If a segment is deemed as more similar to FP than to TP, then it is removed from the set of detected lipids.

The obtained set is the set of detected lipids.

### Example 3 - Detecting TCFA on the set of images with detected lipids, obtained from Example 2.

The OCT image was blurred with a linear blur.

The blurred image and its lipid segmentation was transformed into polar coordinates centered at the center of the image (corresponding to the center of the probe, which is the source of light for an OCT scan). The image was also cut in such a way, so that it contained a constant length of the light ray, starting from the edge of lumen (vessel wall) and going into the vessel.

Next up, an approximate location of fibrous cap is calculated for each row in which a lipid was present, using two algorithms:
a) "Triangle Thresholding", a method which was described above within this document.
b) "Gradient Guessing".

In the case of the "Gradient Guessing" algorithm, the following methodology was used: On a given A-line in the image, a brightest pixel was localized. Next-up, we look for the first pixel after the brightest pixel whose brightness is below 80% of the brightest one, and the last pixel for which the brightness is less than 70% i.e., pixels whose values are defined as the edges of the range 0.75 ± 0.05. This range corresponds to the range, within which we expect to find the fibrous cap border. For further calculations, the center of the region defined by this range was taken as a potential spot for locating the fibrous cap border.

Resulting means were combined with the distance of lumen border alongside each light ray (i.e., for each row), resulting in values that informs how far from the image center the potential fibrous cap border is.

A Savitzky-Golay filter was applied, to smoothen the datapoints using a cubic polynomial, with the following limitations:
- If there was more than 60 datapoints (36-degree lipid arc), window of size 36 was used.
- For the range of 24-60 datapoints, window of size 24 was used.
- For the range of 4-24 datapoints, window size was equal to the dataset size.
- If there were 3 or less datapoints, smoothing was not conducted.

Smoothed values are used as the fibrous cap border. All marked lipids that are closer to the probe than the border are considered to be part of the fibrous cap.

For further proceedings, a TCFA definition was used where TCFA is define as fibrous cap with minimal thickness < 65 µm. Such a value is considered a threshold for fibrous cap thickness, underneath which a lipid plaque with its fibrous cap is considered a TCFA. This value was entered into the algorithm.

Thickness of the fibrous cap was defined by measuring the distance between the fibrous cap border and the edge of lumen.

Verifying whether a plaque is a TCFA by comparing it with the aforementioned threshold value. When thickness drops under 65 µm across any A-line, a plaque is considered a TCFA.

Provided results are compared with doctors, reaching accuracy of 76.53% for all analyzed OCT image frames.

### Example 4 - Detecting the location fibrous cap for different ratios of weights for the "Gradient Guessing" and "Triangle Thresholding" algorithms

In order to determine the optimal ratio of weights for the "Gradient Guessing" (GG) and "Triangle Thresholding" (TT) algorithms, a variety of ratios were studied using the results from example 3 in order to find a ratio, for which the fibrous cap border detection is most accurate.

On Figure 6 a result for the following three ratios is presented:

| | GG | TT |
|---|---|---|
| White line | 0.75 | 0.25 |
| Light gray line | 0.10 | 0.90 |
| Dark gray line | 0.90 | 0.10 |

As one can see, for all proportions a good approximation of the fibrous cap within the lipid was obtained (lipid angle marked with a gray shade). Results of the light gray and dark gray lines are closely aligned to the results of "Triangle Thresholding" and "Gradient Guessing" respectively, however they are not equivalent to those algorithms. The closest match was obtained when ratio of GG:TT was equal 0.75:0.25, represented by a white line.

## Claims

1. Method of automatically detecting and segmenting TCFA on Optical Coherence Tomography (OCT) images, made up of the following steps:
a) downloading OCT image data, which are to be analyzed in order to detect and segment TCFA, which contain at least one image on which there is at least one lipid plaque,
where said at least one lipid plaque is segmented and marked for analysis;
b) calculating an approximate location for the border cap thickness through independent processing of two algorithms within the aforementioned at least one lipid plaque marked for analysis, alongside every light ray of the scan (A-line), where said algorithms are:
∘ "Gradient Guessing" and
∘ "Triangle Thresholding";
after which a weighted mean is applied to the two above algorithms, where the weights are between 0.60 and 0.90 for the "Gradient Guessing" algorithm, and between 0.10 and 0.40 for the "Triangle Thresholding" algorithm, where the weights are selected so that they sum to 1;
c) smoothing the image to obtain a fibrous cap border within the aforementioned at least one lipid plaque marked for analysis;
d) determining the thickness of the fibrous cap via the shortest distance between the fibrous cap border and the vessel wall;
e) determining whether a given lipid plaque is a TCFA or not, marking it as TCFA if the result is positive,
where lipid plaque is labeled as TCFA, when the minimum thickness of the fibrous cap does not exceed a given threshold value for fibrous cap thickness;
where, in case of the OCT image data containing more than one lipid plaque, stages b)-e) are conducted for every lipid plaque, and all of the above is conducted automatically, without human input.

2. Method from claim 1, in case the OCT image data has more than one lipid plaque, stages b)-e) are done in sequence or simultaneously for each of the lipid plaques.

3. Method from claim 1 or 2, in which the OCT image data has the aforementioned at least one lipid plaque marked via its lipid angle, in stage e) the lipid plaque is marked as TCFA, when fibrous cap thickness does not exceed a fibrous cap thickness threshold value, and the aforementioned lipid angle exceeds a given lipid angle threshold value.

4. Method according to any of the claims 1-3, in which "Gradient Guessing" contains the following steps:
localizing the brightest pixel for each A-line, which is defined as maximum light intensity for a given A-line,
localizing series of neighboring pixels that are further from lumen than the brightest pixel, for which the brightness value is between 65% and 85%, ideally within 70% to 80%, with respect to the maximum light intensity or a given A-line,
marking the center of the aforementioned series of pixels as the first potential location for the fibrous cap border,
Where the above steps are done for every A-line within the lipid plaque marked for analysis.

5. Method according to any of the claims 1-4, in which "Triangle Thresholding" algorithm contains the following steps:
obtaining a histogram of pixel intensities for a given image,
marking two points on the histogram: first point (A), on the top of the highest peak, as well as a second point (B), representing edge value belonging to the darkest pixel,
marking on the histogram a third point (C) which is between the two points above, such that the area of the triangle ABC is maximized,
calculating the distance (d) between the points of crossing the histogram's OX axis of lines that are perpendicular to the OX axis, and which pass through first point (A) and second point (B),
marking the point of crossing the histogram's OX axis of a third line, passing through the third point (C) perpendicularly to the OX axis,
defining point (P) existing a third of the length of (d) from the point of crossing of the third line with the OX axis, and assuming the histogram value of that point as the threshold value,
marking pixels on the OCT image, for which the intensity values are above the threshold value, as a second potential location for fibrous cap border.

6. Method according to any of the claims 1-5, in which in step b) used weights are from 0.65 to 0.85 for the "Gradient Guessing" algorithm, and between 0.15 and 0.35 for "Triangle Thresholding" algorithm, and which ideally are 0.75 for "Gradient Guessing" algorithm and 0.25 for "Triangle Thresholding" algorithm.

7. Method according to any of the claims 1-6, in which in step c) to smooth the results a Savitzky-Golay filter was used.

8. Method according to claim 7, using the following smoothing conditions:
if the amount of datapoints is larger than 60 (i.e., lipid angle is at least 36 degrees), then the smoothing window size is equal 36,
if the amount of datapoints is in the range of 24 to 60 (i.e., the lipid angle is from 14.4 to 36 degrees), a smoothing window of size 24 is used,
if the number of datapoints is in the range of 4 to 24 (i.e., the lipid angle is from 1.8 to 14.4 degrees), then the smoothing window has size equal to the number of datapoints, and
if the number of datapoints is less than or equal 3 (i.e., lipid angle is at most 1.8 degrees) then no smoothing is conducted.

9. Method according to any of the claims 1-8, in which on step e) a given threshold value for cap thickness is set in-advance by the user prior to the execution of the method.

10. Method according to any of the claims 1-9, in which on step e) marking of a given lipid plaque as TCFA for a positive result is done in any way allowing for visual recognition of TCFA.

11. Method according to claim 10, in which the marking of a given lipid plaque as TCFA is done via a method allowing for visual recognition via projecting the image into a reconstruction of the vessel, with the results being recognizably layered on top alongside the detected TCFA directly in Augmented Reality (AR) and/or in Mixed Reality (MR).

12. Set-up for automatic detecting and marking TCFA on Optical Coherence Tomograph (OCT) images signified by at least one processor configured to:
a) download OCT image data, which are to be analyzed in order to detect and mark TCFA, whereas the aforementioned data contains at least one image with at least one lipid plaque, where said at least one lipid plaque is marked for analysis;
b) calculating an approximate location of the fibrous cap border via independent calculation of two algorithms within aforementioned at least one lipid plaque marked for analysis, along each of the scan's light rays (A-lines), where the aforementioned algorithms are:
∘ "Gradient Guessing" and
∘ "Triangle Thresholding"
and then calculating a weighted mean for the two algorithms above, where the used weights used between 0.60 and 0.90 for the "Gradient Guessing" algorithm, and
between 0.10 and 0.40 for "Triangle Thresholding" algorithm, where they are selected so that they sum to 1;
c) smoothing of the resulting fibrous cap border within the aforementioned at least one lipid plaque marked for analysis;
d) calculating the thickness of the fibrous cap by marking the shortest distance between the fibrous cap border and the vessel wall;
e) deciding, whether a given lipid plaque is a TCFA or not, and marking it as TCFA if said decision is positive,
where the lipid plaque is marked as TCFA, when the thickness of the fibrous cap does not exceed the threshold value for fibrous cap thickness;
Where in a case where on OCT image data there is more than one lipid plaque, steps b)-e) are done for each of the lipid plaques.

13. Set-up according to claim 12, which contains at least one user interface configured for inputting to the processor a threshold value for the thickness of the fibrous cap, used in step e).

14. Set-up according to claims 12 or 13, which contains at least one imaging module configured to showcasing the result of segmentation of TCFA plaques obtained in step e).

15. A data carrier, **characterized by** containing a computer software which causes the processor to execute a method for automatic detecting and marking of TCFA on Optical Coherence Tomograph (OCT) images, described in any of the claims 1-11.

## Amended claims

### Amended claims in accordance with Rule 137(2) EPC.

1. Method of automatically detecting and segmenting TCFA on Optical Coherence Tomography (OCT) images, made up of the following steps:
a) downloading OCT image data, which are to be analyzed in order to detect and segment TCFA, which contain at least one image on which there is at least one lipid plaque, and which contain segmentation of lumen, the boundaries of which correspond to vessel wall,
where said at least one lipid plaque is segmented and marked for analysis;
b) calculating an approximate location for the fibrous cap border through independent processing of two algorithms within the aforementioned at least one lipid plaque marked for analysis, alongside every light ray of the scan (A-line), where said algorithms are:
∘ "Gradient Guessing" and
∘ "Triangle Thresholding";
wherein Gradient Guessing" comprises the following steps:
localizing the brightest pixel for each A-line, which is defined as maximum light intensity for a given A-line,
localizing series of neighboring pixels that are further from lumen than the brightest pixel, for which the brightness value is between 65% and 85%, ideally within 70% to 80%, with respect to the maximum light intensity or a given A-line,
marking the center of the aforementioned series of pixels as the first potential location for the fibrous cap border,
Where the above steps are done for every A-line within the lipid plaque marked for analysis, and
wherein "Triangle Thresholding" algorithm comprises the following steps:
obtaining a histogram of pixel intensities for a given image,
marking two points on the histogram: first point (A), on the top of the highest peak, as well as a second point (B), representing edge value belonging to the darkest pixel,
marking on the histogram a third point (C) which is between the two points above, such that the area of the triangle ABC is maximized,
calculating the distance (d) between the points of crossing the histogram's OX axis of lines that are perpendicular to the OX axis, and which pass through first point (A) and second point (B),
marking the point of crossing the histogram's OX axis of a third line, passing through the third point (C) perpendicularly to the OX axis,
defining point (P) existing a third of the length of (d) from the point of crossing of the third line with the OX axis, and assuming the histogram value of that point as the threshold value,
marking pixels on the OCT image, for which the intensity values are above the threshold value, as a second potential location for fibrous cap border;
after which a weighted mean is applied to the approximate locations predicted by the two above algorithms, where the weights are between 0.60 and 0.90 for the "Gradient Guessing" algorithm, and between 0.10 and 0.40 for the "Triangle Thresholding" algorithm, where the weights are selected so that they sum to 1;
c) smoothing the image to obtain a fibrous cap border within the aforementioned at least one lipid plaque marked for analysis;
d) determining the thickness of the fibrous cap via the shortest distance between the fibrous cap border and the vessel wall;
e) determining whether a given lipid plaque is a TCFA or not, marking it as TCFA if the result is positive,
where lipid plaque is labeled as TCFA, when the minimum thickness of the fibrous cap does not exceed a given threshold value for fibrous cap thickness;
where, in case of the OCT image data containing more than one lipid plaque, stages b)-e) are conducted for every lipid plaque, and all of the above is conducted automatically, without human input.

2. Method according to claim 1, in case the OCT image data has more than one lipid plaque, stages b)-e) are done in sequence or simultaneously for each of the lipid plaques.

3. Method according to claim 1 or 2, in which the OCT image data has the aforementioned at least one lipid plaque marked via its lipid angle, in stage e) the lipid plaque is marked as TCFA, when fibrous cap thickness does not exceed a fibrous cap thickness threshold value, and the aforementioned lipid angle exceeds a given lipid angle threshold value.

4. Method according to any of the claims 1-3, in which in step b) used weights are from 0.65 to 0.85 for the "Gradient Guessing" algorithm, and between 0.15 and 0.35 for "Triangle Thresholding" algorithm, and which ideally are 0.75 for "Gradient Guessing" algorithm and 0.25 for "Triangle Thresholding" algorithm.

5. Method according to any of the claims 1-4, in which in step c) to smooth the results a Savitzky-Golay filter was used.

6. Method according to claim 5, using the following smoothing conditions:
if the amount of datapoints is larger than 60 (i.e., lipid angle is at least 36 degrees), then the smoothing window size is equal 36,
if the amount of datapoints is in the range of 24 to 60 (i.e., the lipid angle is from 14.4 to 36 degrees), a smoothing window of size 24 is used,
if the number of datapoints is in the range of 4 to 24 (i.e., the lipid angle is from 1.8 to 14.4 degrees), then the smoothing window has size equal to the number of datapoints, and
if the number of datapoints is less than or equal 3 (i.e., lipid angle is at most 1.8 degrees) then no smoothing is conducted.

7. Method according to any of the claims 1-6, in which on step e) a given threshold value for cap thickness is set in-advance by the user prior to the execution of the method.

8. Method according to any of the claims 1-7, in which on step e) marking of a given lipid plaque as TCFA for a positive result is done in any way allowing for visual recognition of TCFA.

9. Method according to claim 8, in which the marking of a given lipid plaque as TCFA is done via a method allowing for visual recognition via projecting the image into a reconstruction of the vessel, with the results being recognizably layered on top alongside the detected TCFA directly in Augmented Reality (AR) and/or in Mixed Reality (MR).

10. System for automatic detecting and marking TCFA on Optical Coherence Tomograph (OCT) images signified by at least one processor configured to:
a) download OCT image data, which are to be analyzed in order to detect and mark TCFA, whereas the aforementioned data contains at least one image with at least one lipid plaque, and which contain segmentation of lumen, the boundaries of which correspond to vessel wall,
where said at least one lipid plaque is marked for analysis;
b) calculating an approximate location of the fibrous cap border via independent calculation of two algorithms within aforementioned at least one lipid plaque marked for analysis, along each of the scan's light rays (A-lines), where the aforementioned algorithms are:
∘ "Gradient Guessing" and
∘ "Triangle Thresholding";
wherein Gradient Guessing" comprises the following steps:
localizing the brightest pixel for each A-line, which is defined as maximum light intensity for a given A-line,
localizing series of neighboring pixels that are further from lumen than the brightest pixel, for which the brightness value is between 65% and 85%, ideally within 70% to 80%, with respect to the maximum light intensity or a given A-line,
marking the center of the aforementioned series of pixels as the first potential location for the fibrous cap border,
Where the above steps are done for every A-line within the lipid plaque marked for analysis, and
wherein "Triangle Thresholding" algorithm comprises the following steps:
obtaining a histogram of pixel intensities for a given image,
marking two points on the histogram: first point (A), on the top of the highest peak, as well as a second point (B), representing edge value belonging to the darkest pixel,
marking on the histogram a third point (C) which is between the two points above, such that the area of the triangle ABC is maximized,
calculating the distance (d) between the points of crossing the histogram's OX axis of lines that are perpendicular to the OX axis, and which pass through first point (A) and second point (B),
marking the point of crossing the histogram's OX axis of a third line, passing through the third point (C) perpendicularly to the OX axis,
defining point (P) existing a third of the length of (d) from the point of crossing of the third line with the OX axis, and assuming the histogram value of that point as the threshold value,
marking pixels on the OCT image, for which the intensity values are above the threshold value, as a second potential location for fibrous cap border;
and then calculating a weighted mean for the approximate locations predicted by the two algorithms above, where the used weights used between 0.60 and 0.90 for the "Gradient Guessing" algorithm, and between 0.10 and 0.40 for "Triangle Thresholding" algorithm, where they are selected so that they sum to 1;
c) smoothing of the resulting fibrous cap border within the aforementioned at least one lipid plaque marked for analysis;
d) calculating the thickness of the fibrous cap by marking the shortest distance between the fibrous cap border and the vessel wall;
e) deciding, whether a given lipid plaque is a TCFA or not, and marking it as TCFA if said decision is positive,
where the lipid plaque is marked as TCFA, when the thickness of the fibrous cap does not exceed the threshold value for fibrous cap thickness;
Where in a case where on OCT image data there is more than one lipid plaque, steps b)-e) are done for each of the lipid plaques.

11. System according to claim 10, which contains at least one user interface configured for inputting to the processor a threshold value for the thickness of the fibrous cap, used in step e).

12. System according to claims 10 or 11, which contains at least one imaging module configured to showcasing the result of segmentation of TCFA plaques obtained in step e).

13. A data carrier, **characterized by** containing a computer software which causes the processor to execute a method for automatic detecting and marking of TCFA on Optical Coherence Tomograph (OCT) images, described in any of the claims 1-9.
